Europäisches Patentamt

European Patent Office

Office européen des brevets

(11) Publication number: **0 299 746 B1**

# EUROPEAN PATENT SPECIFICATION

(45) Date of publication of patent specification: **26.08.92**   (51) Int. Cl.⁵: **C07K 3/18**, C12N 15/27

(21) Application number: **88306421.4**

(22) Date of filing: **13.07.88**

Consolidated with 88906521.5/0368904
(European application No./publication No.) by
decision dated 06.08.90.

(54) **Purification of GM-CSF.**

(30) Priority: **16.07.87 US 74410**

(43) Date of publication of application:
**18.01.89 Bulletin 89/03**

(45) Publication of the grant of the patent:
**26.08.92 Bulletin 92/35**

(84) Designated Contracting States:
**AT BE CH DE ES FR GB GR IT LI LU NL SE**

(56) References cited:
**EP-A- 0 183 198**
**EP-A- 0 215 126**
**WO-A-86/00639**

(73) Proprietor: **SCHERING CORPORATION**
**2000 Galloping Hill Road**
**Kenilworth New Jersey 07033(US)**

(72) Inventor: **Trotta, Paul P.**
**405 Park Avenue**
**Rutherford New Jersey 07070(US)**
Inventor: **Reichert, Paul**
**11 Cambray Road**
**Montville New Jersey 07045(US)**
Inventor: **Seelig, Gail F.**
**130 Coriell Avenue**
**Fanwood New Jersey 07023(US)**
Inventor: **Kosecki, Robert A.**
**29 White Birch Lane**
**Parsippany New Jersey 07054(US)**

(74) Representative: **Ritter, Stephen David et al**
**Mathys & Squire 10 Fleet Street**
**London EC4Y 1AY(GB)**

**Description**

SUMMARY OF THE INVENTION

The present invention relates to a method for purifying granulocyte-macrophage colony-stimulating factor (GM-CSF), particularly human GM-CSF.

The detection, isolation and purification of these factors is extremely difficult, for it is frequently complicated by the nature of the supernatants in which they are typically located, the divergencies and cross-overs of activities of the various components in the mixtures, the sensitivity (or lack thereof) of the assays utilized to ascertain the factors' properties, the frequent similarity in the range of molecular weights and other characteristics of the factors, and the very low concentration of the factors in their natural settings.

The method of this invention provides for a particular series of purification steps which result in GM-CSF with high specific biological activity, high electrophoretic purity and a low level of pyrogens or other contaminants.

BACKGROUND

Circulating blood cells are constantly replaced by newly developed cells. Replacement blood-cells are formed in a process termed hematopoiesis, in which at least eight mature blood cell lineages are produced: red blood cells (erythrocytes), macrophages (monocytes), eosinophilic granulocytes, megakaryocytes (platelets), neutrophilic granulocytes, basophilic granulocytes (mast cells), T lymphocytes, and B lymphocytes [see Burgess and Nicola, Growth Factors and Stem Cells (Academic Press, New York, 1983)]. Much of the control of blood cell formation is mediated by a group of interacting glycoproteins termed colony stimulating factors (CSFs). These glycoproteins are so named because of the in vivo and in vitro assays used to detect their presence. Techniques for the clonal culture of hematopoietic cells in semisolid culture medium have been especially important in the development of in vitro assays. In such cultures, individual progenitor cells (i.e., cells developmentally committed to a particular lineage, but still capable of proliferation) are able to proliferate to form a colony of maturing progeny in a manner which is believed to be essentially identical to the comparable process in vivo.

As more CSFs become available, primarily through molecular cloning, interest has heightened in finding clinical applications for them. Their use has been suggested for several clinical situations where the stimulation of blood cell generation would be desirable, such as for rehabilitative therapy after chemotherapy or radiation therapy of tumors, treatment of myeloid hypoplasias, treatment of neutrophil deficiency, treatment to enhance hematopoietic regeneration following bone marrow transplantation, and treatment to increase host resistance to established infections.

Complementary DNAs (cDNAs) for GM-CSF, factors which support growth and development of granulocytes and macrophages, have recently been cloned and sequenced by a number of laboratories. Moreover, non-recombinant GM-CSF has been purified from culture supernatants of the Mo cell line (described in U.S. Patent 4,438,032), and the first sixteen amino acids from the N-terminus have been sequenced [Gasson et al., Science, Vol. 226, pgs. 1339-1342 (1984)]. Among the human GM-CSFs, heterogeneity of the nucleotide sequence and amino acid sequence has been observed. For example, at the amino acid level both threonine and isoleucine have been observed at position 100 with respect to the N-terminal alanine, suggesting that several allelic forms, or polymorphs, of GM-CSF may exist within human populations.

A variety of methods are now available for de novo preparation and cloning of cDNAs, and for the construction of cDNA libraries.

By way of example, total mRNA is extracted from cells (e.g., a nontransformed human T cell source) producing polypeptides exhibiting the desired activity. The double-stranded cDNAs can be constructed from this total mRNA by using primer-initiated reverse transcription to make first the complement of each mRNA sequence, and then by priming for second strand synthesis. Subsequently, the cDNAs can be cloned by joining them to suitable plasmids or bacteriophage vectors through complementary homopolymeric tails or cohesive ends created with linker segments containing appropriate restriction sites, and then transforming a suitable host. A wide range of expression systems (i.e., host/expression-vector combinations) can be used to produce the proteins purified by the process of this invention. Possible types of host cells include, but are not limited to, bacterial, yeast, insect, mammalian and the like.

Various methods have been disclosed for extracting the GM-CSF from the host cells and subsequently purifying it, but GM-CSF is not always adequately purified in good yield and with retention of biological activity.

## DETAILED DESCRIPTION

The present invention comprises a unique series of chromatographic steps which purify GM-CSF to more than 95% homogeneity, with the retention of biological activity, and in good yield.

The procedure is composed of sequential anion-exchange, dye-ligand affinity, gel filtration and reversed-phase chromatography.

The anion exchange column is used to remove proteases from the supernatant obtained after the host cells are killed and filtered off. Quaternary aminoethyl, mixed amine or other intermediate base resins or weak base resins such as p-amino benzyl cellulose are particularly useful. Quaternary aminoethyl is a preferred anion exchange resin. The quaternary aminoethyl may be attached to a cross-linked dextran, cellulose, agarose or acrylic support.

The dye-ligand affinity column is used to remove hydrophobic impurities. A typical column comprises a triazinyl dye such as Procion Red HE-3B on an agarose support (e.g., Matrex Gel Red A, manufactured by Amicon Corp., Scientific Systems Division, Danvers, MA).

Gel filtration is used to separate high and low molecular weight impurities. A particularly useful filtration gel is a cross-linked dextran-based gel, identified by the trademark SEPHADEX G-100, manufactured by Pharmacia Fine Chemicals (Piscataway, N.J.). The resin has a fractionation molecular weight range of 4,000 to 150,000 for globular proteins and peptides and 1,000 to 100,000 polysaccharides. Other commercially available resins having cut-off ranges of from about 1,000 to about 200,000 for proteins may also be used.

Reversed phase chromatography, e.g. fast protein liquid chromatography (FPLC) and high pressure liquid chromatography (HPLC), has been found to be the only procedure capable of removing GM-CSF-related proteins identifiable on electrophoresis with no apparent denaturation of GM-CSF. Any C4 or C8 column may be used, for example, PRORPC-C8 (manufactured by Pharmacia) for FPLC and Dynamax-C4 or Dynamax-C8 (manufactured by Rainin Instruments Co., Woburn, MA) for HPLC.

In particular the present invention relates to the purification of proteins having the sequence of the cDNA insert of clone pcD-human-GM-CSF illustrated below, which has been deposited with the American Type Culture Collection, Rockville, Maryland, under accession number 39923.

## HUMAN GM-CSF

```
         10         20         30                        47
ACACAGAGAG AAAGGCTAAA GTTCTCTGGA GG ATG TGG CTG CAG AGC CTG CTG CTC TTG
                                    MET Trp Leu Gln Ser Leu Leu Leu Leu

   62             77                  92                107
GGC ACT GTG GCC TGC AGC ATC TCT GCA CCC GCC CGC TCG CCC AGC CCC AGC ACG
Gly Thr Val Ala Cys Ser Ile Ser Ala Pro Ala Arg Ser Pro Ser Pro Ser Thr

       122               137                152                167
CAG CCC TGG GAG CAT GTG AAT GCC ATC CAG GAG GCC CGG CGT CTC CTG AAC CTG
Gln Pro Trp Glu His Val Asn Ala Ile Gln Glu Ala Arg Arg Leu Leu Asn Leu

               182                197                212
AGT AGA GAC ACT GCT GCT GAG ATG AAT GAA ACA GTA GAA GTC ATC TCA GAA ATG
Ser Arg Asp Thr Ala Ala Glu Met Asn Glu Thr Val Glu Val Ile Ser Glu Met

   227                242                257                272
TTT GAC CTC CAG GAG CCG ACC TGC CTA CAG ACC CGC CTG GAG CTG TAC AAG CAG
Phe Asp Leu Gln Glu Pro Thr Cys Leu Gln Thr Arg Leu Glu Leu Tyr Lys Gln

           287                302                317
GGC CTG CGG GGC AGC CTC ACC AAG CTC AAG GGC CCC TTG ACC ATG ATG GCC AGC
Gly Leu Arg Gly Ser Leu Thr Lys Leu Lys Gly Pro Leu Thr Met Met Ala Ser

332                347                362                377
CAC TAC AAG CAG CAC TGC CCT CCA ACC CCG GAA ACT TCC TGT GCA ACC CAG ATT
His Tyr Lys Gln His Cys Pro Pro Thr Pro Glu Thr Ser Cys Ala Thr Gln Ile

       392                407                422                437
ATC ACC TTT GAA AGT TTC AAA GAG AAC CTG AAG GAC TTT CTG CTT GTC ATC CCC
Ile Thr Phe Glu Ser Phe Lys Glu Asn Leu Lys Asp Phe Leu Leu Val Ile Pro


           452              467       477       487       497
TTT GAC TGC TGG GAG CCA GTC CAG GAG TGA GACCGGCCAG ATGAGGCTGG CCAAGCCGGG
Phe Asp Cys Trp Glu Pro Val Gln Glu  .

      507        517        527        537        547        557
GAGCTGCTCT CTCATGAAAC AAGAGCTAGA AACTCAGGAT GGTCATCTTG GAGGGACCAA

      567        577        587        597        607        617
GGGGTGGGCC ACAGCCATGG TGGGAGTGGC CAGGACCTGC CCTGGGCACA CTGACCCTGA

      627        637        647        657        667        677
TACAGGCATG GCAGAAGAAT GGGAATATTT TATACTGACA GAAATCAGTA ATATTTATAT

      687        697        707        717        727        737
ATTTATATTT TTAAAATATT TATTTATTTA TTTATTTAAG TTCATATTCC ATATTTATTC

      747        757        767        777        787
AAGATGTTTT ACCGTAATAA TTATTATTAA AAATATGCTT CTAAAAAAAA
```

The following are general aspects of the purification protocol, which is described in more detail in seven stages below:

All operations are performed at 2-15°C, unless otherwise indicated. Protein concentration is determined at each stage by a Coomassie blue binding assay. pH measurements may vary by ± 0.2 pH units and conductivity measurements may vary by ± 3 mS. If the expected degree of purification is not achieved in any chromatographic procedure, eluted fractions may be rechromatographed on the same type of column or, alternatively, recycled through a previous step or a previous series of steps. An ammonium sulfate precipitation and/or ultrafiltration step may be performed to concentrate and/or store the protein, as e.g. in Stage III below. All solvents, including those that are employed for either equilibration or elution of columns, are filtered prior to use through a 0.2 $\mu$ membrane. USP XIX grade water is employed in the preparation of all solvents after Stage IV below.

Stage I. Cell Killing and Protein Extractions

Cells are killed, for example by adjusting to pH 2.0 (or less) by sequential additions of suitable acids, e.g. by adding 85% phosphoric acid to pH 4.5 and 50% trichloroacetic acid to pH 2.0. Substantially all of the suspension liquid is removed from the cells and discarded, a second suspension of the treated cells is prepared, said second suspension is neutralized, and the GM-CSF-containing liquid is separated from the suspended cells, e.g. by filtration through a 0.2 $\mu$ membrane. The conductivity of the extract is then adjusted to 15-20 mS by addition of water. A low conductivity is required to minimize the amount of dilution required to allow GM-CSF to bind to the QAE column.

Stage II. Quaternary Aminoethyl (QAE) Column Chromatography

If required, the neutralized extract is adjusted to pH 7.5 with sodium hydroxide or hydrochloric acid, as appropriate. The conductivity of the neutralized extract is adjusted to 5-10 mS by addition of deionized water. The QAE column or other suitable anion exchange column is equilibrated with at least 2-3 column volumes of suitable pH 7.5 buffer, e.g. 20 mM Tris-HCl. The extract containing GM-CSF is applied to the column at a loading of not greater than 20 mg per ml of resin. The column is eluted with 10-20 column volumes of a gradient of sodium chloride in the range of 0-0.5 M dissolved in the same buffer in which the column was equilibrated. Sodium chloride or other appropriate salt is added to increase the conductivity and thereby elute the GM-CSF. Fractions are combined based on sodium dodecyl sulfate-polyacrylamide gel electrophoresis (SDS-PAGE) and chromatographed in Stage III.

Stage III. Dye Affinity Chromatography

The combined fractions from the anion exchange (e.g. QAE) column are diluted with deionized water to a conductivity of 5-10 mS and loaded onto a dye-ligand affinity column, e.g. Procion Red HE3B attached to an agarose support, which had been previously equilibrated with 2-3 column volumes of appropriate pH 7.5 buffer, e.g. 20 mM Tris-HCl. The amount of protein loaded onto the column should not exceed 10 mg per ml of gel.

The column is washed with 3-4 column volumes of the equilibration buffer. Elution is performed with between 5 and 15 volumes of a gradient of sodium chloride or other appropriate salt from 0 to 0.75 M dissolved in the equilibration buffer. The fractions to be employed in Stage IV are combined on the basis of SDS-PAGE.

Stage IV. Concentration By Ultrafilitration and/or Ammonium Sulfate Precipitation

If the protein concentration of the combined fractions from the previous stage is less than 1.0 mg/ml, the solution is concentrated by ultrafiltration employing a 3,000-5,000 molecular weight cut-off membrane. The final protein concentration should be 1.0 mg/ml or greater. Ammonium sulfate is added to a final concentration of 60-70%. The precipitate is collected by centrifugation and then washed once with 20 mM Tris-HCl, pH 7.5, or other suitable buffer containing the same concentration of ammonium sulfate as employed for precipitation. The precipitate is collected by centrifugation and dissolved in the same buffer.

Stage V. Gel Filtration Chromatography

The redissolved ammonium sulfate precipitate may be clarified by centrifugation, if required. The solution is filtered through a 0.2 $\mu$ filter and charged onto a gel filtration column such as a Sephadex G-100 column equilibrated with an appropriate buffer at pH 7.5, e.g. 20 mM Tris-HCl. The amount of protein loaded onto the column should not exceed 3 mg per ml of gel. The column is eluted with the same buffer. Fractions for stage VI are combined based on SDS-PAGE.

## Stage VI. Reversed-Phase Column Chromatography

The combined fractions from the gel filtration are passed through a 0.2 $\mu$ filter and applied to a reversed-phase column, e.g., FPLC or HPLC columns. The column is equilibrated with an acidic solution such as 0.1% trifluoroacetic acid (TFA) and elution is performed with a gradient of 0-100% of an inert solvent such as acetonitrile dissolved in the acidic solution. Fractions are combined based on SDS-PAGE. The solution is lyophilized and the dried powder is dissolved in a buffer solution at pH 7.2, e.g., 20 mM sodium phosphate.

## Stage VII. Dialysis of the Purified GM-CSF

The solution from Stage VI is dialyzed against a buffer at pH 7.2, e.g., 20 mM sodium phosphate. Two changes of buffer are performed with a minimum of 4-5 hours between changes. If required, the dialyzed solution is concentrated by ultrafiltration using a 3,000-5,000 molecular weight cut-off membrane to a protein concentration of at least 1.0 mg/ml. The solution of purified GM-CSF is passed through a 0.2 $\mu$ filter and stored frozen at about -20°C or below.

Following is a specific example of GM-CSF purification. Roman numerals correspond to some of the Stages of the general description above.

## Stage II. QAE Column Chromatography

The neutralized protein extract was adjusted to pH 7.5 with sodium hydroxide. The extract was then diluted approximately 2-fold with cold deionized water to reach a final conductivity of 8 mS. A 6.5 liter column (BIOPROCESS 252/15, manufactured by Pharmacia) was equilibrated with 3 column volumes of 20 mM Tris.HCl, pH 7.5. Extract (50 liters) was applied to the column at a loading of 8.6 mg protein per ml of resin. The column was eluted at a flow of 400 ml/min, first with 30 liters of equilibration buffer, and then with 70 liters of a linear gradient established between 35 liters of 20 mM Tris.HCl, pH 7.5 and 35 liters of 20 mM Tris.HCl, pH 7.5 containing 0.4 M NaCl. Fractions (4-5 liters) were combined based on gel electrophoresis (SDS-PAGE) and the pooled protein (2.0 grams) was chromatographed in Stage III.

## Stage III. Dye Affinity Chromatography

The combined fractions from the QAE column were concentrated by ultrafiltration (3,000-5,000 molecular weight cut-off membrane) to 1 liter, and then diluted 5-fold with cold deionized water to a conductivity of 8 mS. This pool (5 liters) was then loaded at a ratio of 6.7 mg/ml onto a 300 ml Matrex Gel Red A column which had previously been equilibrated with three column volumes of 20 mM Tris.HCl, pH 7.5. The column was then washed with 600 ml of equilibrating buffer, and the 2.0 gram load was eluted using a linear gradient established between 1.5 liters of 20 mM Tris.HCl, pH 7.5 and 1.5 liters of 20 mM Tris.HCl, pH 7.5 containing 0.75 M NaCl. The fractions were combined based on gel electrophoresis, and the fractions (1.5 liters containing 1.12 gram of protein) were concentrated by ultrafiltration in Stage IV.

## Stage IV. Concentration of Dye Ligand Eluate By Ultrafiltration

The 1.5 liter eluate of the dye ligand chromatography contained 0.75 mg/ml. Ultrafiltration of this pool was performed in an Amicon high-output stainless steel stirred cell (Model 2000). A YM-5 membrane (150 mm) (Amicon) was used with a flow rate of 10 ml/min., 80 PSI and 4°C. The sample was concentrated to a volume of 75 ml and 10-15 mg/ml. Following the ultrafiltration, the sample was filtered through a .22 $\mu$ cellulose nitrate membrane. Ammonium sulfate was added to a final concentration of 60% (390 mg/ml). The solution was held at 4°C without mixing for 2 hours, and then centrifuged at 4,000 rpm for 45 min at 4°C. The precipitate was collected, and resuspended in one liter of 20 mM Tris.HCl, pH 7.5 containing 60% saturated ammonium sulfate at 4°C. The sample was centrifuged at 4,000 rpm for 45 min at 4°C.

## Stage V. Sephadex G-100 Chromatography

The redissolved ammonium sulfate pellet was charged onto a 2 liter column at a flow rate of 2 ml/min. A loading ratio of 0.5 mg of protein per ml of resin was used and the sample was eluted with the equilibrating buffer, 20 mM Tris.HCl, pH 7.5. Fractions were combined based on gel electrophoresis (SDS-PAGE), and the 300 ml volume containing 350 mg of protein was frozen at -20°C until it was applied to the reversed-phase column.

Stage VI. Reversed-Phase Column Chromatography

The combined fractions of the gel filtration column were passed through a 0.2 μ filter and applied onto a 20 ml PRORPC-C2/C8 (Pharmacia) column at a flow rate of 2.5 ml/min. The column had previously been equilibrated with solvent containing 0.1% TFA in water. Four identical chromatographies (100 mg per run) were performed. Sample was eluted using a linear gradient between 35% acetonitrile, 0.1% TFA and 100% acetonitrile, 0.1% TFA over a 125 min. time period. Fractions were combined based on SDS-PAGE. The solution was lyophilized, and the dried powder was dissolved in 20 mM sodium phosphate, pH 7.2.

Stage VII. Dialysis of The Purified GM-CSF

The pool from the reversed-phase column was dialysed against three changes of 20 mM sodium phosphate buffer, pH 7.2. The solution was then sterile-filtered through a 0.2 μ filter and frozen at -20°C.

## Claims

1. A method of purifying granulocyte-macrophage colony-stimulating factor (GM-CSF) from a culture medium of a microorganism obtained by a recombinant DNA technique and capable of producing the GM-CSF, comprising the sequential steps of removal of proteases by anionexchange chromatography, removal of hydrophobic impurities by dye-ligand affinity chromatography, separation of high and low molecular weight impurities by gel-filtration chromatography, and separation of related proteins by reversed phase chromatography.

2. A method as claimed in claim 1 wherein the GM-CSF is human GM-CSF.

3. A method as claimed in claim 2 wherein the GM-CSF is recombinant human GM-CSF.

4. A method as claimed in any of claims 1 to 3 wherein the anion exchange resin is a quaternary aminoethyl resin.

5. A method as claimed in any of claims 1 to 4 wherein the dye-ligand affinity column comprises a triazinyl dye on an agarose support.

6. A method as claimed in any of claims 1 to 5 wherein the gel-filtration column is a cross-linked dextran-based gel having a cut-off range of from about 1,000 to about 200,000 for proteins.

7. A method as claimed in any of claims 1 to 6 wherein the reversed phase chromatography is fast protein liquid chromatography using a C4 or C8 column.

8. A method as claimed in claim 1 comprising removal of protease with a quaternary aminoethyl resin, removal of hydrophobic impurities with a column comprising a triazinyl dye on an agarose support, removal of high and low molecular weight impurities with a crosslinked dextran-based gel having a cut-off range of from about 1,000 to about 200,000 for proteins, and removal of related proteins by fast protein liquid chromatography using a C4 or C8 column.

## Patentansprüche

1. Verfahren zur Reinigung eines Granulocyten-Makrophagen-Kolonie-stimulierenden Faktors (GM-CSF) aus einem Kulturmedium eines Mikroorganismus, der durch eine DNA-Rekombinationstchnik erhalten wurde und zur Erzeugung des GM-CSF befähigt ist, umfassend die sequentiellen Schritte der Entfernung von Proteasen durch Anionenaustausch-Chromatographie, der Entfernung hydrophober Verunreinigungen durch Farbstoff-Liganden-Affinitäts-Chromatographie, der Abtrennung hochmolekularer und niedermolekularer Verunreinigungen durch Gelfiltrations-Chromatographie und der Abtrennung verwandter Proteine durch Phasenumkehr-Chromatographie.

2. Verfahren nach Anspruch 1, worin der GM-CSF Human-GM-CSF ist.

3. Verfahren nach Anspruch 2, worin der GM-CSF rekombinierter Human-GM-CSF ist.

4. Verfahren nach irgendeinem der Ansprüche 1 bis 3, worin das Anionenaustausch-Harz ein quaternäres Aminoethyl-Harz ist.

5. Verfahren nach irgendeinem der Ansprüche 1 bis 4, worin die Farbstoff-Liganden-Affinitäts-Säule einen Triazinyl-Farbstoff auf einem Agarose-Träger umfaßt.

6. Verfahren nach irgendeinem der Ansprüche 1 bis 5, worin die Gelfiltrations-Säule ein vernetztes Gel auf Dextran-Basis mit einem Ausschlußbereich von etwa 1 000 bis etwa 200 000 für Proteine ist.

7. Verfahren nach irgendeinem der Ansprüche 1 bis 5, worin die Phasenumkehr-Chromatographie eine schnelle Protein-Flüssigkeits-Chromatographie unter Verwendung einer C4-oder C8-Säule ist.

8. Verfahren nach Anspruch 1, umfassend die Entfernung von Protease mittels eines quaternären Aminoethyl-Harzes, die Entfernung hydrophober Verunreinigungen mit Hilfe einer Säule, die einen Triazinyl-Farbstoff auf einem Agarose-Träger umfaßt, die Entfernung hochmolekularer und niedermolekularer Verunreinigungen durch ein vernetztes Gel auf Dextran-Basis mit einem Ausschlußbereich von etwa 1 000 bis etwa 200 000 für Proteine und die Entfernung verwandter Proteine durch schnelle Protein-Flüssigkeits-Chromatographie unter Verwendung einer C4- oder C8-Säule.

## Revendications

1. Méthode de purification du facteur stimulant les colonies de granulocytes-macrophages (GM-CSF) d'un milieu de culture d'un micro-organisme obtenu par une technique d'ADN recombinant et capable de produire GM-CSF, comprenant les étapes séquentielles d'enlèvement des protéases par chromatographie par échange d'anions, d'enlèvement des impuretés hydrophobes par chromatographie par affinité ligand-colorant, de séparation des impuretés de fort et faible poids moléculaire par chromatographie par filtration sur gel et séparation des protéines en rapport par chromatographie en phase inverse.

2. Méthode selon la revendicaiton 1, où GM-CSF est GM-CSF humain.

3. Méthode selon la revendication 2, où GM-CSF est GM-CSF humain recombinant.

4. Méthode selon l'une quelconque des revendications 1 à 3, où la résine d'échange d'anions est une résine d'aminoéthyle quaternaire.

5. Méthode selon l'une quelconque des revendications 1 à 4, où la colonne par affinité ligand-colorant comprend un colorant de triazinyle sur un support d'agarose.

6. Méthode selon l'une quelconque des revendications 1 à 5, où la colonne de filtration sur gel est un gel à base de dextrane réticulé ayant une plage de coupure comprise entre environ 1.000 et environ 200.000 pour les protéines.

7. Méthode selon l'une quelconque des revendications 1 à 6, où la chromatographie en phase inverse est une chromatographie liquide rapide des protéines en utilisant une colonne C4 ou C8.

8. Méthode selon la revendication 1, comprenant l'élimination de la protéase avec une résine d' aminoéthyle quaternaire, l'élimination des impuretés hydrophobes avec une colonne comprenant un colorant de triazinyle sur un support d'agarose, l'éliminaiton des impuretés de faible et fort poids moléculaire avec un gel à base de dextrane réticulé ayant une plage de coupure d'environ 1.000 à environ 200.000 pour les protéines et l'élimination des protéines en rapport par chromatographie liquide rapide des protéines en utilisant une colonne C4 ou C8.